# EUROPEAN PATENT APPLICATION

(11) **EP 4 674 421 A1**
(43) Date of publication of application: **07.01.2026**
(21) Application number: 24764002.2
(22) Date of filing: 29.02.2024
(51) Int. Cl.: A61K 35/76, A61K 38/19, A61K 39/395, A61K 48/00, A61P 35/00

(54) **ANTICANCER AGENT, PHARMACEUTICAL COMPOSITION FOR CANCER TREATMENT, KIT, AND ACTIVATOR**

(30) Priority: 01.03.2023 US 202363449055 P
(71) Applicant: Biocomo Incorporation, Mie, 510-1233 (JP); Mie University, Tsu-shi, Mie 514-8507 (JP)
(72) Inventor: FUKUMURA Masayuki, Mie-gun, Mie 510-1233 (JP); OHTSUKA Junpei, Mie-gun, Mie 510-1233 (JP); MATSUKAWA Yasuhisa, Mie-gun, Mie 510-1233 (JP); NOSAKA Tetsuya, Tsu-shi, Mie 514-8507 (JP); OHTSUKA Tomomi, Tsu-shi, Mie 514-8507 (JP)
(74) Representative: Ipsilon
(86) International application number: PCT/JP2024/007475
(87) International publication number: WO 2024/181525

(57) **Abstract**

An anticancer agent for solid cancer contains, as an active ingredient, human parainfluenza virus type 2 expressing: at least one kind of cytokine selected from the group consisting of IL-2, IL-7, IL-12, IL-15, and IL-18; or a combination of the cytokine and CCL 19 and/or CCL21; or a mutant of either the cytokine or the combination, the mutant having the same function.

## Description

### TECHNICAL FIELD

The present invention relates to an anticancer agent, a pharmaceutical composition for cancer treatment, a kit, and an activator.

### BACKGROUND ART

Cancer cells arise from various abnormalities in the mechanism of immunosuppression, the mechanism of cell proliferation inhibition, gene mutations and translocations, metabolism, and the like. In terms of cancer treatment, the response rate for hematological cancers such as leukemia has increased due to immune checkpoint inhibitors such as anti-PD1 antibodies and CAR-T therapy. (See, for example, Patent Document 1).

### Citation List

### Patent Documents

Patent Document 1: Published Japanese Translation No. 2019-523301 of the PCT International Publication
Patent Document 2: Japanese Patent No. 5801202
Patent Document 3: Japanese Patent No. 5807917
Patent Document 4: Japanese Patent No. 7398680
Patent Document 5: Japanese Patent No. 6358706

### Non Patent Documents

Non Patent Document 1: Hum Gene Ther., (2013) 24(7):683
Non Patent Document 2: Scientific Reports (2023) 13:11361
Non Patent Document 3: Scientific Reports (2019) 9:13999

### SUMMARY OF INVENTION

### Technical Problem

However, it is not easy to increase the response rate for solid cancers using only immune checkpoint inhibitors that inhibit negative immunity and CAR-T therapy. In solid cancers, due to defective angiogenesis, stressful conditions such as hypoxia and a glucose starvation state lead to the formation of a specific microenvironment (tumor microenvironment (TME)) of solid cancers, fibroblasts, macrophages, inflammatory cells, and the like, such as aggravation of cancer cells or drug resistance. Under such circumstances, it has been reported that the infiltration of tumor infiltrating lymphocytes such as CAR-T cells into cancer tissues is inhibited, and the tumor regression effect is reduced. Furthermore, it has also been reported that in humans, the infiltration of immune cells into tumor tissues is hindered by the formation of a matrix of cells and the like between solid cancers and their marginal cells, and that T cell therapy, which is effective in mice, is likely to inhibit infiltration in humans, reducing the antitumor effect.

The present invention has been made in view of the above-described circumstances, and an object of the invention is to provide an anticancer agent, a pharmaceutical composition for cancer treatment, a kit, and an activator, all of which have an excellent antitumor effect.

### Solution to Problem

The present invention includes the following aspects.
[1] An anticancer agent for solid cancer, containing, as an active ingredient, human parainfluenza virus type 2 expressing:
   at least one kind of cytokine selected from the group consisting of IL-2, IL-7, IL-12, IL-15, and IL-18; or
   a combination of the cytokine and CCL19 and/or CCL21; or
   a mutant of either the cytokine or the combination, the mutant having the same function.
[2] The anticancer agent for solid cancer according to [1], in which the at least one kind of cytokine is IL-12 constituting a heterodimer, or a mutant having the same function.
[3] The anticancer agent for solid cancer according to [1], in which the human parainfluenza virus type 2 is of non-proliferative type in which an F gene is deleted from a genome.
[4] A pharmaceutical composition for cancer treatment against solid cancer, the pharmaceutical composition containing the anticancer agent for solid cancer according to any one of [1] to [3].
[5] The pharmaceutical composition for cancer treatment against solid cancer according to [4], further containing an immune activator and/or an immune checkpoint inhibitor.
[6] The pharmaceutical composition for cancer treatment against solid cancer according to [5], in which the immune checkpoint inhibitor includes at least one kind selected from the group consisting of an anti-PD1 antibody, an anti-PD-L1 antibody, an anti-CTLA4 antibody, and an anti-TIM3 antibody.
[7] The pharmaceutical composition for cancer treatment against solid cancer according to [4], in which the pharmaceutical composition for cancer treatment is used for intratumoral administration.
[8] A kit for use in treatment of solid cancer, the kit including, for simultaneous use or sequential use in the treatment:
   human parainfluenza virus type 2 expressing at least one kind of cytokine selected from the group consisting of IL-2, IL-7, IL-12, IL-15, and IL-18, or a combination of the cytokine and CCL19 and/or CCL21, or a mutant of either the cytokine or the combination, the mutant having the same function; and
   an immune activator and/or an immune checkpoint inhibitor.
[9] The kit according to [8], in which the at least one kind of cytokine is IL-12 constituting a heterodimer, or a mutant having the same function.
[10] The kit according to [8], in which the human parainfluenza virus type 2 is of non-proliferative type in which an F gene is deleted from a genome.
[11] The kit according to [8], in which the immune checkpoint inhibitor includes at least one kind selected from the group consisting of an anti-PD1 antibody, an anti-PD-L1 antibody, an anti-CTLA4 antibody, and an anti-TIM3 antibody.
[12] The kit according to any one of [8] to [11], in which the kit is used for intratumoral administration.
[13] A tumor-infiltrating T cell and dendritic cell activator, containing, as an active ingredient:
   human parainfluenza virus type 2 expressing at least one kind of cytokine selected from the group consisting of IL-2, IL-7, IL-12, IL-15, and IL-18; or a combination of the cytokine and CCL19 and/or CCL21; or a mutant of either the cytokine or the combination, the mutant having the same function.

### Advantageous Effects of Invention

According to the present invention, an anticancer agent, a pharmaceutical composition for cancer treatment, a kit, and an activator, all of which have an excellent antitumor effect, can be provided.

### BRIEF DESCRIPTION OF DRAWINGS

[FIG. 1] An explanatory diagram of a plasmid for producing BC-PIV/IL-7 alone or with CCL19 or CCL21. Specifically, FIG. 1 is a construction diagram for introducing a desired gene into the NotI or MluI restriction enzyme sequence site of a plasmid for hPIV2 construction from which an F gene has been deleted.
[FIG. 2] FIG. 2 to FIG. 4 are diagrams showing the expression of IL-7, CCL19, and CCL21 in cells transfected with a BC-PIV in which IL-7 has been introduced alone or together with CCL19 or CCL21, the expression being verified by Western blotting. FIG. 2 is a diagram showing the verification of expression using cells transfected with a BC-PIV in which IL-7 has been introduced.
[FIG. 3] A diagram showing the verification of expression using cells transfected with a BC-PIV in which IL-7 and CCL19 have been introduced.
[FIG. 4] A diagram showing the verification of the expression of IL-7 and CCL21 in BC-PIV-infected ells.
[FIG. 5] An explanatory diagram of a plasmid for producing a BC-PIV in which IL-12 genes as a heterodimer have been introduced. EIS is a sequence that regulates gene expression in BC-PIV.
[FIG. 6] FIG. 6 and FIG. 7 are diagrams showing the expression of IL-12 (P35) and IL-12 (P40) in cells infected with BC-PIV in which IL-12 genes as a heterodimer have been introduced, the expression being verified by Western blotting. FIG. 6 is a diagram showing the verification of the expression of P35 and P40 in BC-PIV-transfected cells, in which the expression utilizes the EIS sequence of BC-PIV.
[FIG. 7] A diagram showing the verification of the expression of P35 and P40 in BC-PIV-transfected cells using BC-PIV constructed by introducing P40 and P35 genes into the NotI and MluI restriction enzyme sequence sites.
[FIG. 8] A chart showing the identification of an IL-12 protein (P70) by ELISA.
[FIG. 9] A diagram showing a schedule of an animal test for verifying the antitumor effect of BC-PIV.
[FIG. 10] A graph showing the antitumor effect of IL-7 and CCL19 expressed by BC-PIV and a synergistic effect with an anti-mouse PD1 antibody.
[FIG. 11] A graph showing the antitumor effect of IL-7 and CCL19 expressed by BC-PIV and a synergistic effect with an anti-mouse PD1 antibody.
[FIG. 12] A graph showing the antitumor effect of IL-7 and CCL19 or CCL21 expressed by BC-PIV and a synergistic effect with an anti-mouse PD1 antibody.
[FIG. 13] A graph showing the antitumor effect of IL-7 and CCL19 or CCL21 expressed by BC-PIV and a synergistic effect with an anti-mouse PD1 antibody.
[FIG. 14] A pathological analysis view of treated tumors.
[FIG. 15] A pathological analysis view of treated tumors.
[FIG. 16] A view showing the evaluation of the distribution of T cells in the inside of the tumor and the peripheral part of the tumor using a T cell-specific antibody.
[FIG. 17] A view showing the evaluation of the distribution of CD8-positive T cells in the inside of the tumor and the peripheral part of the tumor using a CD8-specific antibody.
[FIG. 18] A graph showing the antitumor effect of a BC-PIV in which a cytokine (IL-7) and a chemokine (CCL19) have been introduced, against renal cancer RENCA cells and a synergistic effect of an anti-mouse PD1 antibody.
[FIG. 19] A graph showing the antitumor effect of a BC-PIV in which IL-12 has been introduced, and a synergistic effect of an anti-mouse PD1 antibody.
[FIG. 20] A graph showing the antitumor effect of a BC-PIV in which IL-12 has been introduced, and a synergistic effect of an anti-mouse PD1 antibody.

### DESCRIPTION OF EMBODIMENTS

### <<Promotion of uptake of T cells into tumor tissue>>

### [Chemokine and chemokine receptor]

C-C Chemokine receptor 7 (CCR7) is a major homing receptor for collecting T cells, B cells, and dendritic cells to form a functional microenvironment in secondary lymphatic vessels, and is an important regulator for initiating antigen-specific immune responses. There are two kinds of endogenous ligands for CCR7. One is CCL21, which is constitutively expressed in high endothelial venules (HEVs) of secondary lymphoid tissues. It has been reported that the expression is also induced in lymphatic vessels of inflammatory skin tissues. The other is CCL19, which is produced in the T cell region of lymph nodes and is transcytosed from the basement membrane side to the luminal side of HEV and presented. It has been reported that these chemokine ligand 21 (CCL21) and chemokine ligand 19 (CCL19) have two clearly opposite roles in cancer (see Patent Document 2). One is that CCR7 plays an important role in promoting the metastasis of cancer through the lymphatic system, and the correlation between the upregulation of CCR7 and lymph node (LN) metastasis has been reported in pancreatic cancer, breast cancer, esophageal cancer, head and neck cancer, prostate cancer, colorectal cancer, and the like.

On the other hand, it is thought that this relation between CCR7 and CCL19/21 is also involved in the regulation of immune responses to growing tumors; dendritic cells (DC), CD4-positive helper T cells, regulatory T cells, B cells, memory T cells, and the like express CCR7, which is transported through the interaction with CCL19 and CCL21; CCR7 plays an important role in antigen presentation and activation of T cell-mediated responses; and intratumoral introduction of CCL19 and CCL21 enhances immune responses to the tumor, which is useful for cancer immunotherapy.

CCR7 is a G protein-coupled receptor, and such a receptor is usually susceptible to desensitization, and after initial exposure to a ligand, the receptor is not coupled to further G protein activation. Although CCL21 exhibits activity equivalent to that of CCL19 in the activation of G protein signal transduction through CCR7, it is known that CCL21 does not cause receptor desensitization.

### [Cytokine: IL-7]

Regarding IL-7, IL-7 secreted from thymic epithelial cells helps the proliferation of immature T cells and contributes to the development of T cells, while IL-7 secreted from lymphatic endothelial cells contributes to the survival and the maintenance of the number of naive and memory T cells, and the like.

### [Introduction of chemokines and cytokines]

It is thought that the introduction of the above-mentioned cytokine alone can promote the proliferation of CTL cells having an antitumor effect; however, there is still a possibility that the infiltration of CTL cells into a solid tumor may be inhibited by the marginal cell matrix of the solid tumor. In order to enhance the infiltration of tumor-specific CTL cells into the inside of the solid tumor, it is necessary to take measures to actively incorporate CTL cells into cells in the marginal region of the tumor tissue. For this purpose, it is thought that the antitumor immunity is enhanced when CCL19 or CCL21 is ectopically expressed in the inside of the solid tumor as well as in cells with which CTL cells come into contact, and CTL cells expressing CCR7 are actively incorporated into the tumor tissue.

### [Vector (BC-PIV) expressing two kinds of cytokines and chemokines]

The inventors have so far developed a vector, BC-PIV, that can simultaneously carry a gene and a protein using human parainfluenza virus type 2 (see Patent Document 3). BC-PIV can introduce two or more genes and proteins and has been hitherto shown to be effective in an antitumor immune agent in which a ligand protein of TNFRSF is loaded on BC-PIV (see Patent Document 4). Here, it has been verified that the two genes introduced into BC-PIV are expressed in the infected cells, and this time, genes of cytokines and chemokines will also be introduced into BC-PIV to examine the above-mentioned effects. BC-PIV is derived from human parainfluenza virus type 2, the host is human, and it has been reported that human parainfluenza virus type 2 is poorly permissive in mice so that high expression of the introduced genes is not expected; however, it is believed that as long as the effectiveness can be verified in mice, the extrapolability to humans is high.

Furthermore, BC-PIV has been shown to promote the maturation of dendritic cells (see Non Patent Document 1), so that by directly administering BC-PIV to the inside of a tumor, tumor cells that have been killed or damaged are taken up by antigen-presenting cells such as dendritic cells, the antigen is presented to T cells, and proliferation and activation of T cells and the like having a more effective antitumor effect can be promoted.

### [Cytokine: Introduction of IL-12 into BC-PIV]

IL-12 is a heterodimer consisting of 35 kDa and 40 kDa. IL-12 is one of the cytokines, which is produced by dendritic cells, macrophages, and the like, has useful activity such as assisting the maintenance of activated T cells and the expression of killer T cells (CTLs), and also induces the strongest inflammation. Therefore, there is a considerable interest in the possibility that the cytokine may be used for causing inflammation in tumor tissue and increasing the response rate of immunotherapy. On the other hand, it has been found that IL-12 has extremely strong toxicity due to systemic immune activation, and the clinical effect is extremely limited even at the maximum tolerated dose (MTD).

Many solid cancers have an immune-resistant microenvironment in which the infiltration, activation, and effector function of immune cells are inhibited, and therefore, those solid cancers are in a state referred to as cold tumor, which does not respond to immunotherapy with immune checkpoint inhibitors (ICIs), including an anti-PD1 antibody and an anti-CTLA4 antibody.

Thus, it was considered whether it would be possible to introduce IL-12 as a heterodimer consisting of 35 kDa and 40 kDa into BC-PIV and express IL-12 locally within the tumor to cause inflammation in the tumor tissue, thereby increasing the response rate of immunotherapy. In addition, since BC-PIV can introduce two genes, the introduction of two IL-12 subunit genes was also examined.

### <<Expression of cytokines and chemokines in tumor tissue>>

In solid cancers, due to defective angiogenesis, stressful conditions such as hypoxia and glucose starvation state lead to the formation of a specific microenvironment (tumor microenvironment (TME)) of solid cancers, fibroblasts, macrophages, inflammatory cells, and the like, such as aggravation of cancer cells or drug resistance. In addition, the formation of the marginal cell matrix of solid tumors inhibits the infiltration of antitumor immune cells such as CTL cells into solid tumors. Therefore, it is considered that "active" infiltration action of antitumor immune cells such as T cells into solid tumor tissue is necessary.

Lymphocytes such as T cells are each controlled in terms of migration in vivo and homing to specific tissue by specific chemokines and chemokine receptors, and are involved in the formation and homeostasis of the immune system in the living body. Furthermore, the induction of expression of different chemokines under a disease condition causes various pathological conditions through the migration of lymphocytes and the like.

Lymphocytes such as T cells express CCR7, a chemokine receptor. Two kinds of chemokines, CCL19 and CCL21, are shared ligands for CCR7. Due to the relationship of these, homing of T cells and B cells in peripheral blood as well as antigen-sensitized dendritic cells from afferent lymphatic vessels, to secondary lymphoid tissues is induced.

Therefore, it is considered that by ectopically expressing these chemokines not only inside tumors but also in cells at the boundary sites of solid tumor tissues, lymphocytes expressing CCR7, in particular, tumor-specific T cells and CTLs can be recruited to tumor tissues, and the infiltration thereof into tumor tissues can be actively enhanced.

Interleukin 7 (IL-7) is a cytokine essential for differentiation of early lymphocytes, maintenance of mature T cells, and formation of lymphoid organs, and is considered to be a cytokine responsible for maintaining homeostasis of the immune system.

### <Anticancer agent>

In one embodiment, the present invention provides an anticancer agent for solid cancer, containing, as an active ingredient, human parainfluenza virus type 2 expressing: at least one kind of cytokine selected from the group consisting of IL-2, IL-7, IL-12, IL-15, and IL-18; or a combination of the cytokine and CCL19 and/or CCL21; or a mutant of either the cytokine or the combination, the mutant having the same function.

The virus used in the present embodiment is human parainfluenza virus type 2 (hPIV2). From the viewpoint of not producing secondary infective particles, it is preferable that the human parainfluenza virus type 2 is of non-proliferative type in which an F gene having a membrane fusion function is deleted.

Furthermore, the virus used in the present embodiment expresses at least one kind of cytokine selected from the group consisting of IL-2, IL-7, IL-12, IL-15, and IL-18, or a combination of the cytokine and CCL19 and/or CCL21, or a mutant of either the cytokine or the combination, the mutant having the same function.

Examples of the combination of ligands expressed by the virus include combinations of one kind of cytokine, which is each of IL-2, IL-7, IL-12, IL-15, and IL-18; combinations of two kinds of cytokines selected from the group consisting of IL-2, IL-7, IL-12, IL-15, and IL-18; and combinations of three or more kinds of cytokines selected from the group consisting of IL-2, IL-7, IL-12, IL-15, and IL-18. Specific examples thereof include a combination of IL-2 and CCL19 or CCL21, a combination of IL-7 and CCL19 or CCL21, a combination of IL-12 and CCL19 or CCL21, a combination of IL-15 and CCL19 or CCL21, and a combination of IL-18 and CCL19 or CCL21. As will be described later in the Examples, it has been verified that a BC-PIV in which human IL-12 gene has been introduced has an antitumor effect. IL-12 is a heterodimer consisting of 35 kDa and 40 kDa. As will be described later in the Examples, when introducing IL-12 into BC-PIV, it is preferable that the p35 gene and the p40 gene are linked by the EIS sequence (ctctcataatttaagaaaaaatcataggcccggacgggttag (SEQ ID NO: 4)) of hPIV2.

Furthermore, examples of the combination of ligands expressed by the virus also include combinations of the above-described cytokines and CCL19 and/or CCL21. Examples of such a combination include IL-7/CCL19, IL-7/CCL21, IL-12/CCL19, and IL-12/CCL21. As will be described later in the Examples, it has been verified that a BC-PIV in which human IL-7 gene/human CCL-19 gene have been introduced and a BC-PIV in which human IL-7 gene/human CCL-21 gene have been introduced have an antitumor effect.

These mutants having the same function refer to mutants which have an amino acid sequence in which one or several amino acids have been deleted, substituted, inserted, or added in an amino acid sequence constituting a ligand, and which have a signal transduction ability. The number of amino acids deleted, replaced, inserted, or added is preferably 1 to 20, more preferably 1 to 10, and particularly preferably 1 to 5.

Furthermore, the cytokine and/or chemokine used in the present embodiment also includes homologues thereof.

### <Pharmaceutical composition for cancer treatment against solid cancer>

In one embodiment, the present invention contains the above-mentioned anticancer agent. The pharmaceutical composition for cancer treatment against solid cancer of the present embodiment may further contain an immune activator and/or an immune checkpoint inhibitor.

Examples of the immune activator include an agonist antibody of a TNFRSF receptor, and specific examples thereof include an anti-OX40 agonist antibody, an anti-GITR agonist antibody, an anti-CD30 agonist antibody, an anti-4-1 BB agonist antibody, and an anti-CD27 agonist antibody.

Examples of the immune checkpoint inhibitor include an anti-PD1 antagonist antibody, an anti-PD-L1 antagonist antibody, an anti-CTLA4 antagonist antibody, and an anti-TIM3 antagonist antibody.

It is preferable that the pharmaceutical composition for cancer treatment against solid cancer of the present embodiment contains a pharmaceutically acceptable carrier. As the pharmaceutically acceptable carrier, those conventionally used in preparations can be used without particular limitation, and examples thereof include solvents such as sterilized water and physiological saline; binders such as gelatin, corn starch, gum tragacanth, and gum arabic; excipients such as crystalline cellulose; and swelling agents such as alginic acid.

The pharmaceutical composition for cancer treatment against solid cancer of the present embodiment may contain an additive. Examples of the additive include a lubricating agent such as magnesium stearate; a sweetener such as sucrose, lactose, or saccharin; a flavoring agent such as peppermint or akamono oil; a stabilizer such as benzyl alcohol or phenol; a buffering agent such as a phosphate or sodium acetate; a dissolution aid such as benzyl benzoate or benzyl alcohol; an antioxidant; an antiseptic agent; a surfactant; and an emulsifier.

The pharmaceutical composition for cancer treatment against solid cancer of the present embodiment can be formulated by appropriately combining the above-described pharmaceutically acceptable carrier and additives, and mixing them in a generally accepted unit dose form.

The pharmaceutical composition for cancer treatment against solid cancer of the present embodiment may be formulated into a dosage form that is used orally or a dosage form that is used parenterally, and it is preferable that the pharmaceutical composition for cancer treatment against solid cancer is formulated into a dosage form that is used parenterally. Examples of the dosage form that is used orally include a tablet, a capsule, an elixir, and a microcapsule. Examples of the dosage form that is used parenterally include an injectable preparation, an ointment, and a patch, and an injectable preparation is preferred.

Examples of the solvent for an injectable preparation include isotonic solutions containing adjuvants such as physiological saline, glucose, D-sorbitol, D-mannose, D-mannitol, and sodium chloride. The solvent for an injectable preparation may contain an alcohol such as ethanol; a polyalcohol such as propylene glycol or polyethylene glycol; and a nonionic surfactant such as POLYSORBATE 80 (trademark) or HCO-50.

Examples of the administration to patients include an intranasal method, a transbronchial method, an intramuscular method, a percutaneous method, and an oral method, in addition to intraarterial injection, intravenous injection, and subcutaneous injection; however, intratumoral administration is preferred.

The single dose of the pharmaceutical composition for cancer treatment of the present embodiment varies, when administered parenterally, depending on the target of administration, the target organ, the symptom, and the method of administration; however, for example, in the form of an injectable preparation, it is considered that, usually in a case of an adult (assuming a body weight of 60 kg), 1 × 10⁶ BC-PIV particles to 1 × 10¹¹ BC-PIV particles, for example, 1 × 10⁷ BC-PIV particles to 1 × 10¹¹ BC-PIV particles, are administered per day by intravenous injection or local administration centering on the inside of a tumor. Furthermore, the above-described amount may be administered once or in several divided doses per day.

The application target of the pharmaceutical composition for cancer treatment against solid cancer of the present embodiment is not limited as long as the application target is a solid cancer, and examples thereof include breast cancer (such as infiltrating ductal carcinoma, ductal carcinoma in situ, or inflammatory breast cancer), prostate cancer (such as hormone-dependent prostate cancer or hormone-independent prostate cancer), pancreatic cancer (such as pancreatic duct cancer), gastric cancer (such as papillary adenocarcinoma, mucinous adenocarcinoma, or adenosquamous carcinoma), lung cancer (such as non-small cell lung cancer, small cell lung cancer, or malignant mesothelioma), colon cancer (such as gastrointestinal stromal tumor), rectal cancer (such as gastrointestinal stromal tumor), colorectal cancer (such as familial colorectal cancer, hereditary nonpolyposis colorectal cancer, or gastrointestinal stromal tumor), small intestine cancer (such as non-Hodgkin lymphoma or gastrointestinal stromal tumor), esophageal cancer, duodenal cancer, tongue cancer, pharyngeal cancer (such as nasopharyngeal cancer, oropharyngeal cancer, or hypopharyngeal cancer), head and neck cancer, salivary gland cancer, brain tumors (such as pineal astrocytoma, pilocytic astrocytoma, diffuse astrocytoma, or anaplastic astrocytoma), neurinoma, liver cancer (such as primary liver cancer, extrahepatic bile duct cancer), kidney cancer (such as renal cell cancer, transitional cell carcinoma of the renal pelvis and ureter), gallbladder cancer, bile duct cancer, endometrial cancer, cervical cancer, uterine sarcoma, ovarian cancer (such as epithelial ovarian cancer, extragonadal germ cell tumor, ovarian germ cell tumor, or low-grade ovarian tumor), bladder cancer, urethral cancer, skin cancer (such as Merkel cell cancer), hemangioma, melanoma (malignant melanoma), thyroid cancer (such as thyroid medullary carcinoma), parathyroid cancer, nasal cavity cancer, sinus cancer, bone tumors (such as osteosarcoma, Ewing's sarcoma, or soft tissue sarcoma), metastatic medulloblastoma, hemangiofibroma, dermatofibrosarcoma protuberans, retinoblastoma, penile cancer, testicular tumor, pediatric solid cancer (such as Wilms tumor or pediatric renal tumor), Kaposi's sarcoma, Kaposi's sarcoma caused by AIDS, maxillary sinus tumors, fibrous histiocytoma, leiomyosarcoma, rhabdomyoblastoma, and chronic myeloproliferative disease.

### <Kit>

In one embodiment, the present invention provides a kit for use in treatment of cancer, the kit including, for simultaneous use or sequential use in the treatment: human parainfluenza virus type 2 expressing at least one kind of cytokine selected from the group consisting of IL-2, IL-7, IL-12, IL-15, and IL-18, or a combination of the cytokine and CCL19 and/or CCL21, or a mutant of either the cytokine or the combination, the mutant having the same function; and an immune activator and/or an immune checkpoint inhibitor.

In addition, preferred aspects of the kit of the present embodiment are similar to those of <Pharmaceutical composition for cancer treatment>. The order in a case of sequential administration is not particularly limited.

### <Tumor-infiltrating T cell and dendritic cell activator>

In one embodiment, the present invention provides a tumor-infiltrating T cell and dendritic cell activator, containing, as an active ingredient, human parainfluenza virus type 2 expressing: at least one kind of cytokine selected from the group consisting of IL-2, IL-7, IL-12, IL-15, and IL-18; or a combination of the cytokine and CCL19 and/or CCL21; or a mutant of either the cytokine or the combination, the mutant having the same function.

As will be described later in the Examples, in a BC-PIV-administered group loaded with IL-7/CCL19 or IL-7/CCL21, infiltration of T cells inside the tumor and infiltration of lymphocytes in the periphery of the tumor are observed, and an antitumor effect is provided through such infiltration.

### <Treatment method>

In one embodiment, the present invention provides a treatment method for cancer, including administering the above-mentioned anticancer agent or pharmaceutical composition for cancer treatment to a patient in need of treatment.

Furthermore, in one embodiment, the present invention provides a method for treating cancer, including simultaneously or sequentially administering an effective amount of human parainfluenza virus type 2 that expresses a desired cytokine or a desired combination of a cytokine and a chemokine, and an effective amount of an immune activator and/or an immune checkpoint inhibitor to a patient in need of treatment. In addition, preferred aspects of the treatment method of the present embodiment are similar to those of <Pharmaceutical composition for cancer treatment>. The order in a case of sequential administration is not particularly limited.

### [Other embodiments]

In one embodiment, the present invention provides an anticancer agent, a pharmaceutical composition for cancer treatment, or a kit for cancer treatment. As the anticancer agent, the pharmaceutical composition for cancer treatment, or the kit, the same ones as described above can be used.

### Examples

Next, the present invention will be described in more detail by way of Examples; however, the present invention is not intended to be limited to the following Examples.

In the following examples, IL-7, IL-12, CCL19, CCL21, and the like are referred to as being human-derived.

### [Experimental Example 1] Construction of BC-PIV carrying IL-7 gene, CCL19 gene, and CCL21 gene

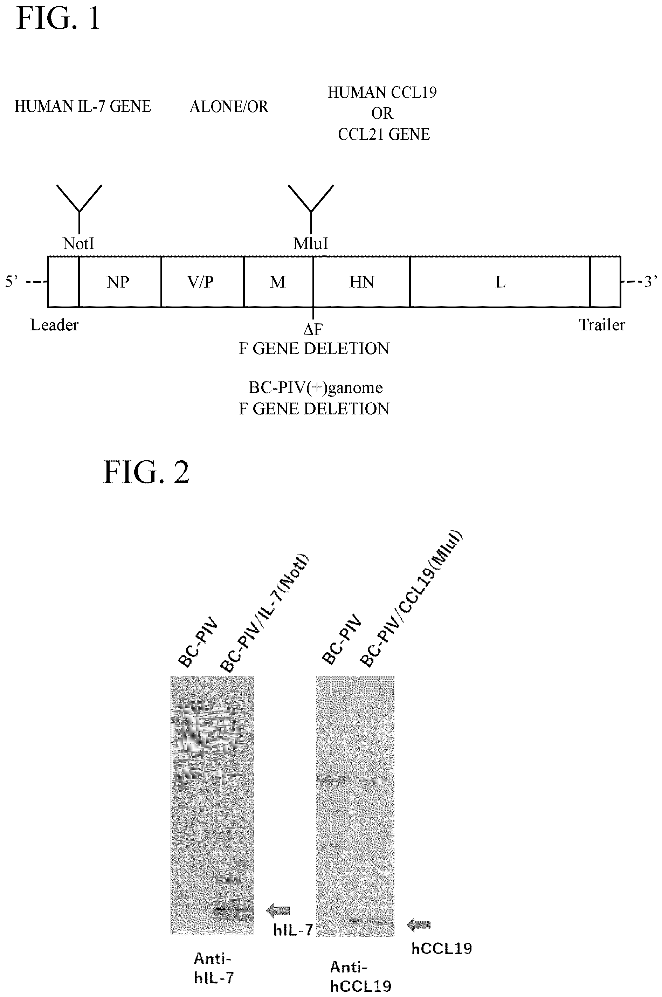

The combinations of plasmids (see FIG. 1) for constructing BC-PIVs, in which IL-7 gene, CCL19 gene, and CCL21 gene have been introduced singly, or two genes into BC-PIV have been introduced, are as follows.

IL-7 gene alone, CCL19 gene alone, a combination of two genes of IL-7/CCL19, and a combination of two genes of IL-7/CCL21 were introduced into BC-PIV (see FIG. 1). These gene sequences are human-derived sequences.

The IL-7 gene sequence used in this case is GenBank: BC047698.1 (SEQ ID NO:1). The CCL19 gene sequence is GenBank: BC027968.1 (SEQ ID NO:2). The CCL21 gene sequence is GenBank: BC027918.1 (SEQ ID NO:3). A gene to which a tag to be incorporated into BC-PIV was added was totally synthesized based on these genes. The collection of BC-PIV from the plasmid was carried out by a reverse genetics method previously used by the inventors (see Patent Documents 4 and 5).

### [Experimental Example 2] Introduction of IL-7, CCL19, and CCL21 genes into BC-PIV and identification of desired gene in infected cells

It was examined whether the BC-PIV collected in Example 1 expressed the target protein. BC-PIV each carrying all the genes introduced into the NotI restriction enzyme sequence site and/or the MluI restriction enzyme sequence site of the plasmid for virus production produced in Example 1 was collected. Next, the collected BC-PIV was used to transfect Vero cells expressing the F membrane protein of hPIV2, which were then cultured for 7 days, and the collected cells were subjected to Western blotting using a commercially available anti-human IL-7 antibody (GeneTex, Cat#: GTX131448), a commercially available anti-human CCL19 antibody (R & D Systems, Cat#: AF361), and a commercially available anti-human CCL21 antibody (company name: Abcam, Cat#: ab9851).

As shown in FIG. 2, expression of each protein was observed in BC-PIV-infected cells in which IL-7 gene was inserted at the NotI restriction enzyme sequence site or in BC-PIV infected cells in which CCL19 gene was inserted at the MluI restriction enzyme sequence site. In the Western blotting, three bands were observed for IL-7. This is considered to be due to differences in glycosylation, as reported.

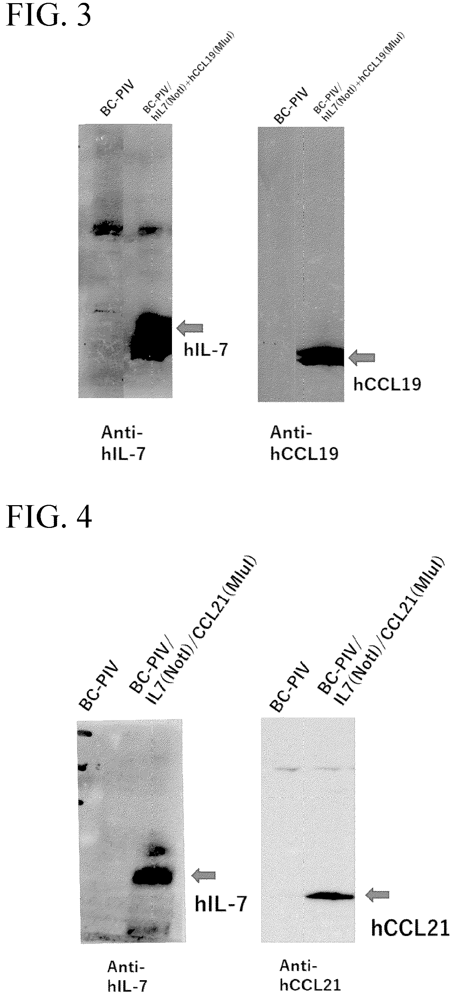

As shown in FIG. 3, expression of both proteins was also observed in a BC-PIV in which two genes, CCL19 and IL-7 genes, had been introduced.

Furthermore, as shown in FIG. 4, in simultaneous introduction of IL-7 gene and CCL21 gene as well, expression of each protein was observed in each gene-intoduced BC-PIV-infected cells.

### [Experimental Example 3] Construction of BC-PIV carrying IL-12 gene

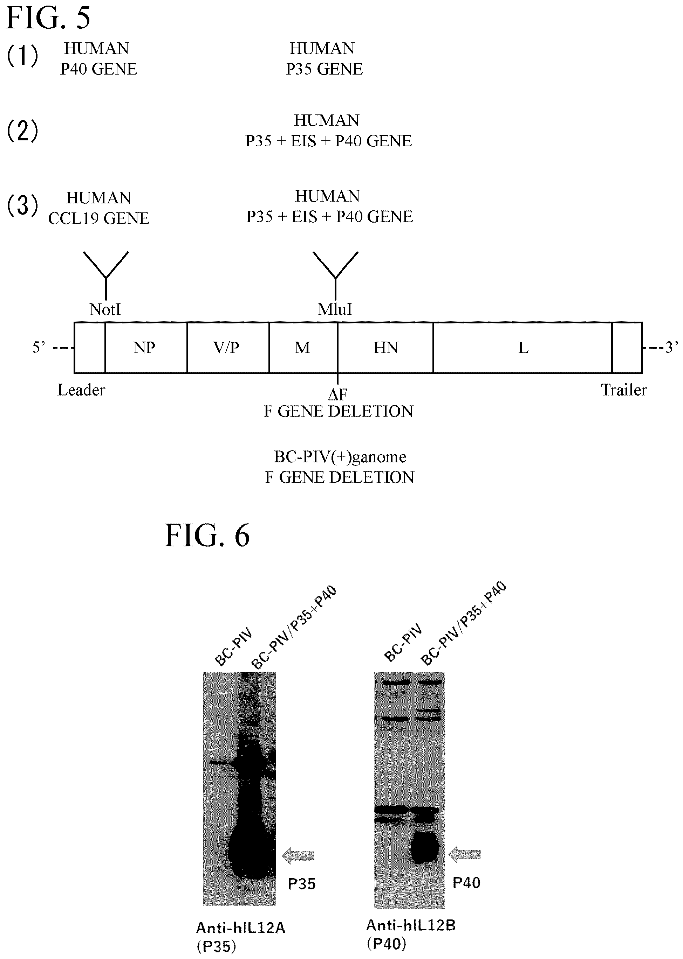

A BC-PIV in which IL-12 gene was introduced was constructed (see FIG. 5). The IL-12 cytokine is a heterodimer (P35 and P40). p35 gene and/or p40 gene was introduced into the NotI restriction enzyme sequence site and the MluI restriction enzyme sequence site of BC-PIV, and the BC-PIV was collected.

Furthermore, in order to combine chemokines and the like in addition to cytokines having two genes consisting of a heterodimer as one set, such as IL-12 cytokine, a BC-PIV in which the p35 gene and the p40 gene were linked by the EIS sequence (ctctcataatttaagaaaaaatcataggcccggacgggttag) (SEQ ID NO:4) of hPIV2 and inserted at the NotI restriction enzyme sequence site, was collected.

The p35 gene sequence of IL-12 used in this case is GenBank: NM_000882.4 (SEQ ID NO:5), and the p40 gene sequence is GenBank: NM_002187.3 (SEQ ID NO:6).

### [Experimental Example 4] Introduction of IL-12 gene into BC-PIV and identification of desired gene in infected cells

It was examined whether the BC-PIV collected in Example 3 expressed the target protein. p35 gene and p40 gene were introduced into the MluI restriction enzyme sequence site of a BC-PIV carrying the p35 and p40 genes, which had been introduced into the NotI restriction enzyme sequence site and/or the MluI restriction enzyme sequence site of the plasmid for virus production produced in Example 3, using the EIS sequence of hPIV2, and the virus was collected (see FIG. 4). The collected BC-PIV was used to infect Vero cells expressing the F membrane protein of hPIV2, which were then cultured for 7 days, and the collected cells were subjected to Western blotting using a commercially available anti-human P35 antibody (Abcam, Cat#: ab131039) and a commercially available anti-human P40 antibody (company name: Abcam, cat#: ab133752). As shown in FIG. 6, P40 and P35 proteins expressed using the EIS of hPIV2 were identified.

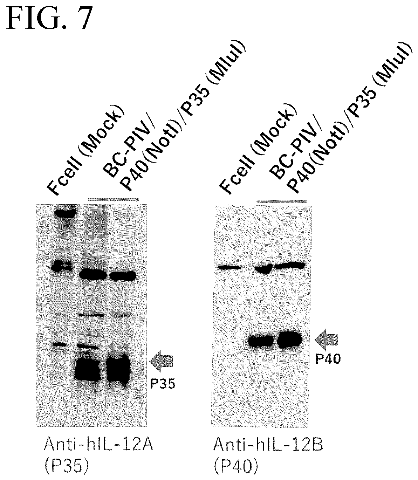

Furthermore, in FIG. 7, the expression of P40 and P35 proteins was verified in cells infected with a BC-PIV which had been collected after introducing P40 gene and P35 gene into the restriction enzyme sequence sites of NotI and MluI shown in FIG. 5.

As a result, it was verified that BC-PIV can simultaneously express P40 and P35 proteins in a single cell using a single vector.

### [Experimental Example 5] Verification of IL-12 expression by ELISA

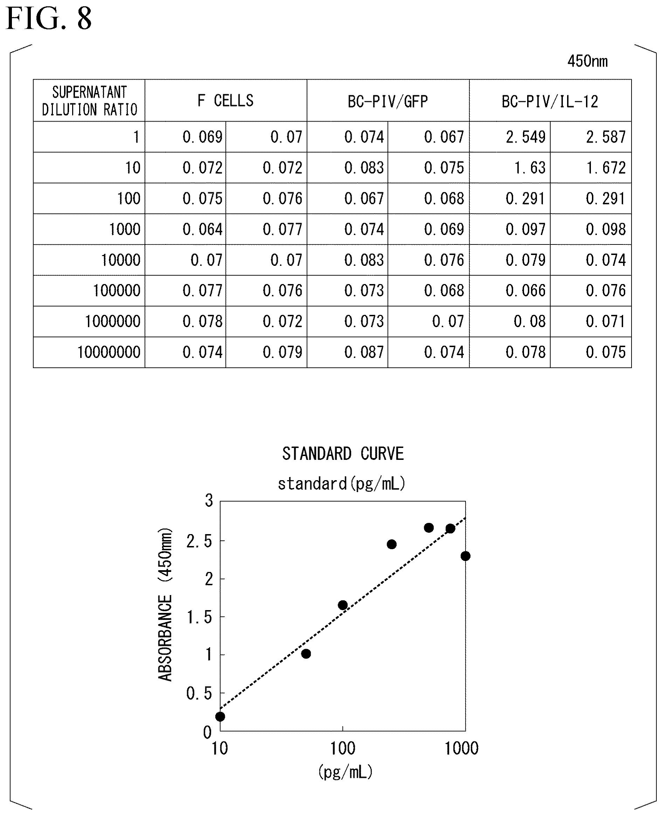

A supernatant of infected cells (4 days after transfection) in which IL-12 was expressed using BC-PIV, was subjected to ELISA. Bio Legend, Cat #: 431704 was used as the ELISA measurement kit. The measurement was carried out according to the measurement method of the kit. The concentration was measured using a P70-specific antibody in the ELISA. From the dilution ratios and the standard curve shown in FIG. 8, expression of IL-12 protein at a concentration of approximately 1.0 ng/mL in the supernatant was verified.

### [Experimental Example 6] Antitumor effect of BC-PIV with cytokine (IL-7) and chemokine (CCL19) introduced therein and synergistic effect of anti-mouse PD1 antibody (first round)

CT26 mouse colorectal cancer cells were transplanted into BALB/c mice using the acquired BC-PIV in which IL-7 gene and CCL19 gene had been introduced, and the antitumor effect was examined. It is known that BC-PIV is poorly permissive for transfection and proliferation in mouse tissues and cells, and when the antitumor effect is observed in the current test, extrapolability to humans can be highly expected.

After removing hair from the ventral side of the mice, 5 × 10⁵ CT26 mouse colorectal cancer cells were subcutaneously transplanted at two sites on the ventral side of each mouse, and 7 days later, the effects of a BC-PIV (Emp) without any exogenous gene introduced therein and an IL-7/CCL19-introduced BC-PIV in a unilateral tumor that had grown to have a tumor diameter of about 5 to 7 mm were examined.

This time, the synergistic effect of the immune checkpoint inhibitor with the anti-mouse PD1 antibody was also examined. It has been reported that the anti-mouse PD1 antibody has a low effect on mouse B16F10 melanoma cells, and evaluation was carried out using CT26 mouse colorectal cancer-transplanted BALB/c mice.

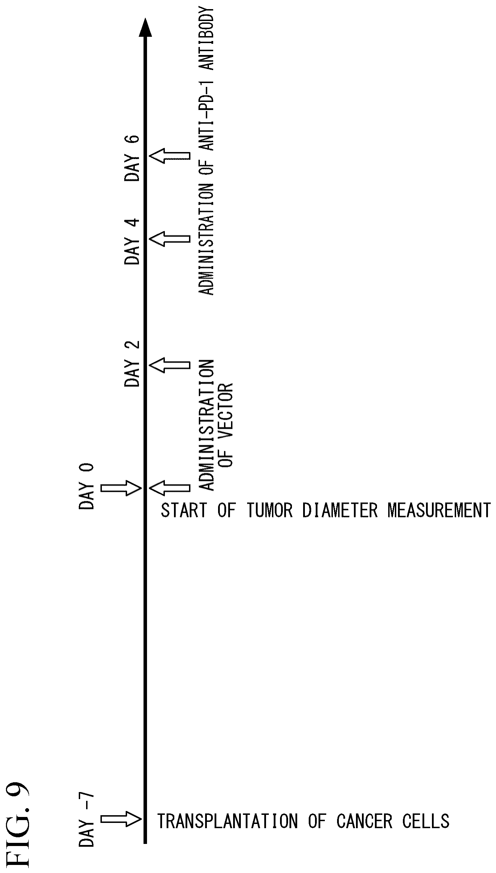

Approximately 1 × 10⁷ TCID₅₀BC-PIVs were administered 7 days and 9 days after the transplantation, and 200 µg of the anti-mouse PD1 antibody was intraperitoneally administered 11 days and 13 days after the transplantation. Thereafter, the tumor diameter was measured every 3 or 4 days, and the tumor volume was calculated by (major axis × minor axis²) ÷ 2. An experimental schedule is shown in FIG. 9.

The group configuration of the mice included the following six groups.
(1): Untreated group (non-treat)
(2): BC-PIV group (Emp)
(3): Anti-mouse PD1 antibody group (αPPD1)
(4): BC-PIV + anti-mouse PD1 antibody group (Emp + αPD1)
(5): BC-PIV/IL-7/CCL19 group (IL-7/CCL19)
(6): BC-PIV/IL-7/CCL19 + anti-mouse PD1 antibody group
(IL-7/CCL19 + αPD1)

### [Experimental Example 7] Antitumor effect of BC-PIV with cytokine (IL-7) and chemokine (CCL19) introduced therein and synergistic effect with anti-mouse PD1 antibody (first round)

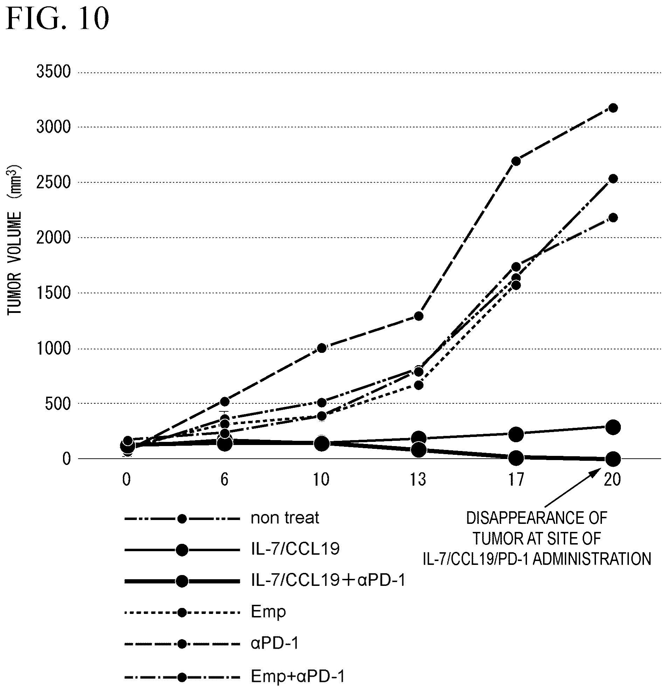

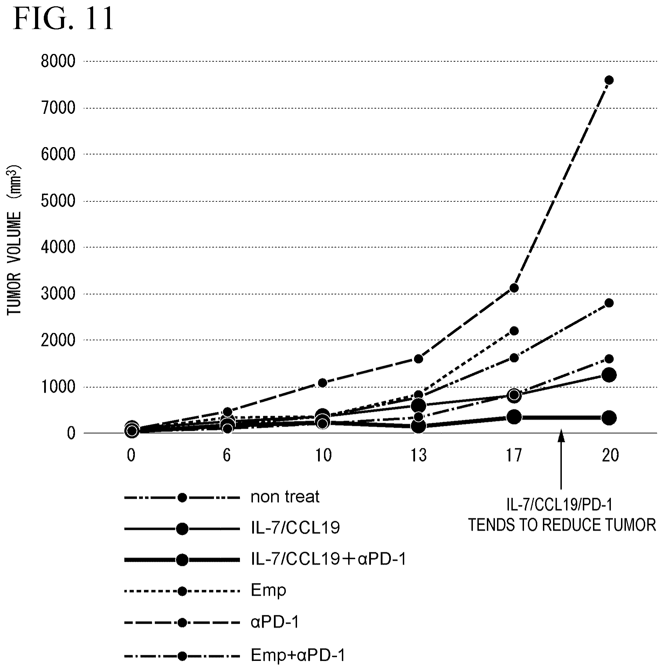

The results are shown in FIG. 10 and FIG. 11. Regarding the tumor volume on the administered side (see FIG. 10), no significant antitumor effect (reduction in tumor volume) was observed in the untreated group, the BC-PIV group (Emp), the anti-mouse PD1 antibody group (αPD1), and the BC-PIV + anti-mouse PD1 antibody group (Emp + αPD1). Furthermore, in the current administration, the antitumor effect of the anti-mouse PD1 antibody alone was hardly observed.

On the other hand, an antitumor effect was observed in the BC-PIV/IL-7/CCL19 group (IL-7/CCL19) and the BC-PIV/IL-7/CCL19 + anti-mouse PD1 antibody group (IL-7/CCL19 + αPD1) (see FIG. 10). On the non-administered side, the BC-PIV/IL-7/CCL19 + anti-mouse PD1 antibody group (IL-7/CCL19 + αPD1) exhibited an antitumor effect, and the abscopal effect was observed (see FIG. 11). In summary, an antitumor effect was observed in the transplanted tumors of the mice in the groups (5) and (6) on the administered side. Even on the non-administered side, a high tumor regression effect was observed in the mice of the group (6), and an even higher abscopal effect was observed due to a synergistic effect with the anti-mouse PD1 antibody.

### [Experimental Example 8] Antitumor effect of BC-PIV with cytokine (IL-7) and chemokine (CCL19 or CCL21) introduced therein and synergistic effect with anti-mouse PD1 antibody (second round)

The test was carried out according to the same technique as in Example 6.

The group configuration of the mice included the following six groups.
(1): Untreated group (non-treat)
(2): BC-PIV group (Emp)
(3): BC-PIV/IL-7/CCL19 group (IL-7/CCL19)
(4): BC-PIV/IL-7/CCL21 group (IL-7/CCL21)
(5): BC-PIV/IL-7/CCL21 + anti-mouse PD1 antibody group
(IL-7/CCL21 + αPD1)

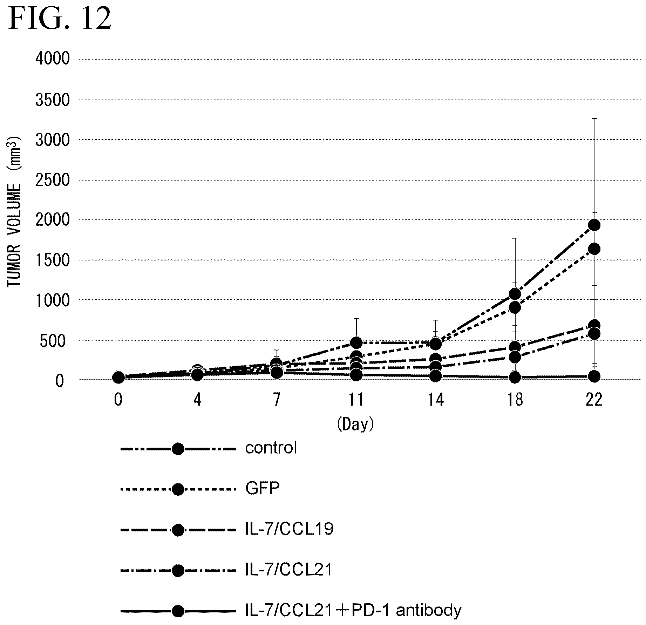

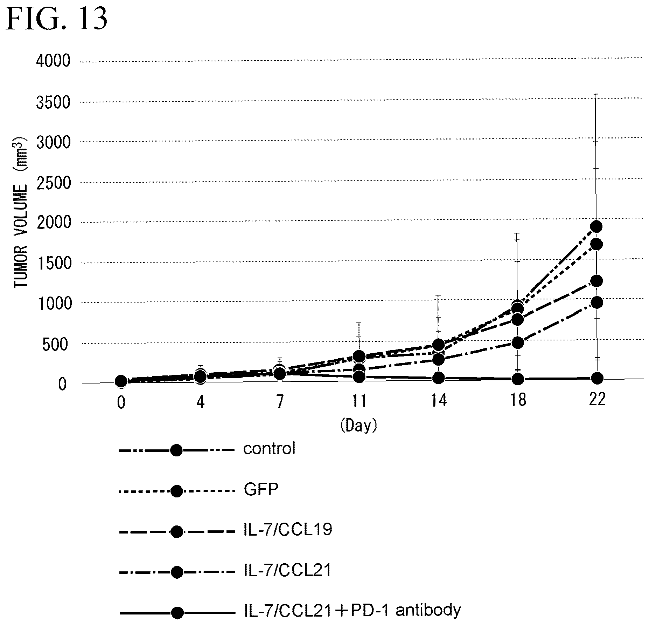

The results are shown in FIG. 12 and FIG. 13. Similarly to the results of Example 8, no significant antitumor effect (reduction in tumor volume) was observed in the untreated group and the BC-PIV group (Emp) in terms of the tumor volume on the administered side (see FIG. 12). On the other hand, an antitumor effect was observed in the BC-PIV/IL-7/CCL19 group (IL-7/CCL19), the BC-PIV/IL-7/CCL21 group (IL-7/CCL21), and the BC-PIV/IL-7/CCL19 + anti-mouse PD1 antibody group (IL-7/CCL19 + αPD1) (see FIG. 12). On the non-administered side, a high antitumor effect was observed in the BC-PIV/IL-7/CCL21 + anti-mouse PD1 antibody group (IL-7/CCL21 + αPD1), and the abscopal effect was observed (see FIG. 13). When CCL19 and CCL21 were compared, CCL21 tended to have a high antitumor effect. It has been reported that although CCL21 exhibits activity equivalent to that of CCL19 in the activation of G protein signal transduction through CCR7, CCL21 does not cause receptor desensitization, and thus, there is a possibility that CCL21 may have a higher antitumor effect.

### [Experimental Example 9] Antitumor effect of BC-PIV with cytokine (IL-7) and chemokine (CCL19 or CCL21) introduced therein, and activation of T cells/B cells of tumor after 8 days from initial administration by treatment with anti-mouse PD1 antibody

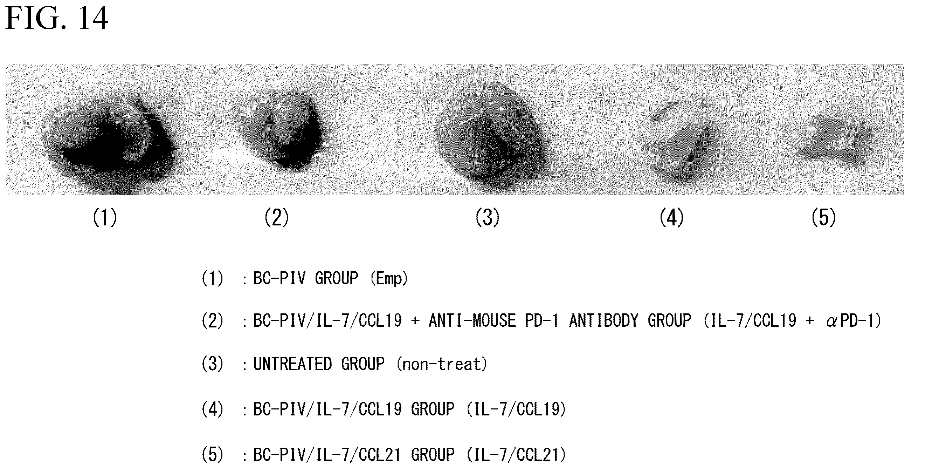

Eleven days after the BC-PIV administration, the treated mice were subjected to perfusion fixation with 4% paraformaldehyde, and tumors on the administered side were excised (see FIG. 14) and subjected to an anti-mouse T cell antibody (company name: CST, cat #: 78588) and an anti-mouse B cell antibody (company name: CST, Cat #: 98941).

The group configuration of the mice included the following five groups.
(1): BC-PIV group (Emp)
(2): BC-PIV/IL-7/CCL19 + anti-mouse PD1 antibody group
   (IL-7/CCL19 + αPD1)
(3): Untreated group (non-treat)
(4): BC-PIV/IL-7/CCL19 group (IL-7/CCL19)
(5): BC-PIV/IL-7/CCL21 group (IL-7/CCL21)

From the state of the size of the tumor mass, the tumor size of the mice to which IL-7/CCL19, IL-7/CCL21, and IL-7/CCL19 + anti-mouse PD1 antibody were administered was small, and it was speculated from the tumor diameter and appearance that there was an antitumor effect.

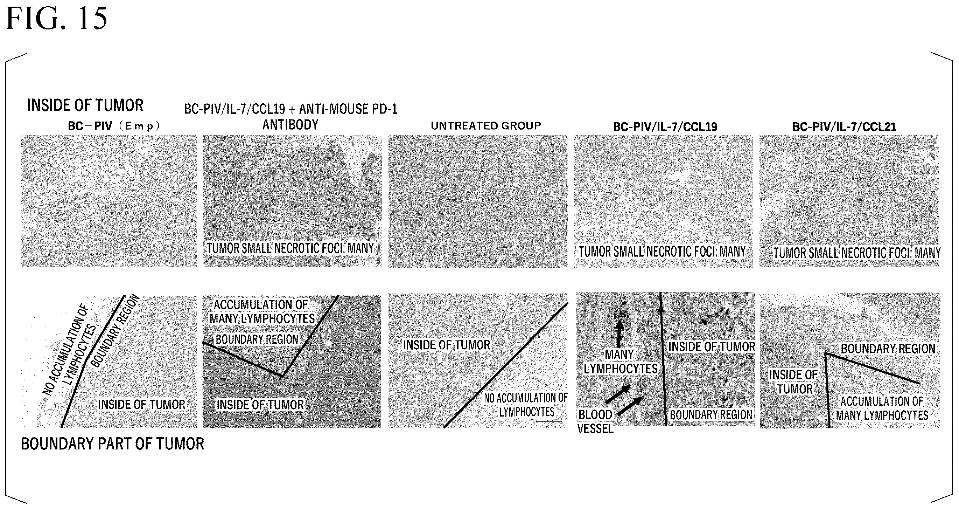

The features of these treated tumors based on an HE staining image (see FIG. 15) were that the necrotic foci of tumor cells inside the tumor were large, and many tumor cells remaining around the necrosis were observed to begin to degenerate. In particular, a feature point is that accumulation of a large number of lymphocytes is observed at the boundary part with the tumor and around the blood vessels outside the tumor. This feature was observed in the BC-PIV/IL-7/CCL19-administered tumors in which a significant increase in lymphocytes was verified around the blood vessels at the tumor boundary part (see FIG. 15).

On the other hand, in the BC-PIV-administered and untreated tumors, infiltration of T cells into the tumor occurred less compared to the groups administered with IL-7/CCL19, IL-7/CCL21, and IL-7/CCL19 + anti-mouse PD1 antibody; however, T cell-positive cells were observed inside the tumor. However, accumulation of lymphocytes in the tumor peripheral region was not observed (see FIG. 15). This was conceived to be due to the accumulation of a large number of lymphocytes expressing the CCR7 receptor around the tumor as a result of the expression of CCL19 or CCL21 chemokine caused by transfection of cells inside the tumor and around the tumor with BC-PIV.

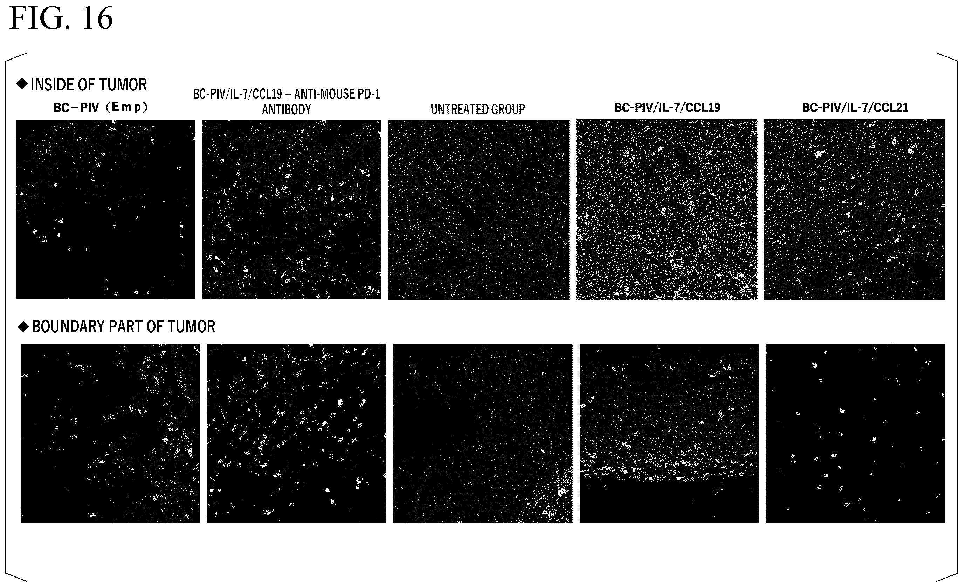

FIG. 16 shows the state of T cells inside the tumor and in the periphery of the tumor. No positive reaction to the anti-mouse B cell antibody was observed in any of the tumors, and it was conceived that there was no large accumulation of B cells caused by the treatment. On the other hand, in the tumors administered with IL-7/CCL19, IL-7/CCL21, and IL-7/CCL19 + anti-mouse PD1 antibody, a large number of T cell-positive cells were observed within the tumor and around the tumor.

The tumors of the mice administered with IL-7/CCL19, IL-7/CCL21, and IL-7/CCL19 + anti-mouse PD1 antibody were smaller; however, infiltration of T cell-positive cells was observed even inside the tumors administered with BC-PIV alone (see FIG. 16). However, infiltration of lymphocytes in the periphery of the tumor was not observed. It has been previously verified that the effect of maturing dendritic cells with single administration of BC-PIV is high (see Non Patent Document 1); however, this time, it was verified that administration of BC-PIV alone can activate T cells but does not have an ability to accumulate these lymphocytes near the administration site.

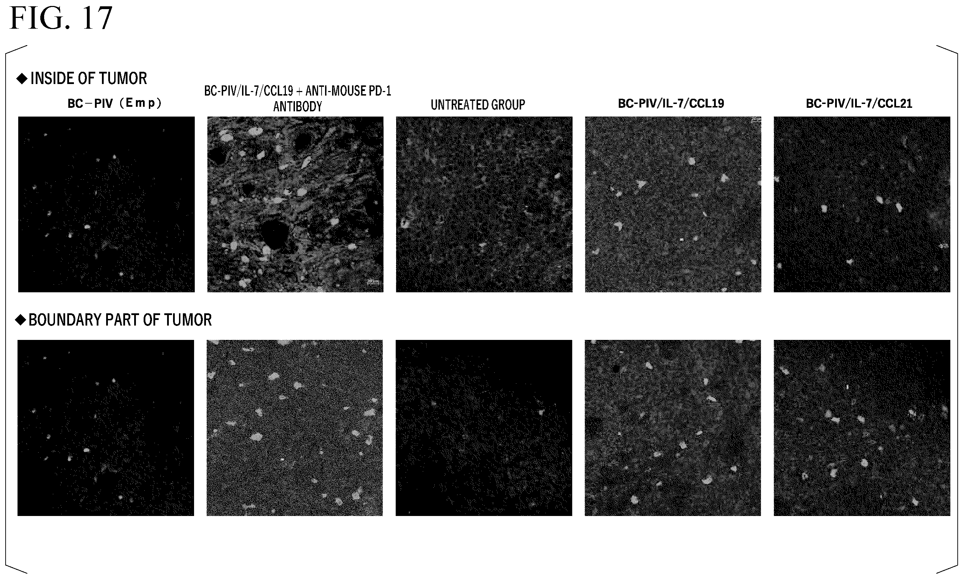

FIG. 17 shows the state of CD8-positive T cells inside the tumor and in the periphery of the tumor. Positive reactions of CD4-positive T cells to the anti-mouse B cell antibody in the inside of the tumor and the peripheral part of the tumor were hardly observed in any of the tumors, and it was conceived that there was no large accumulation of CD4-positive T cells caused by the treatment. On the other hand, in the tumors administered with IL-7/CCL19, IL-7/CCL21, and IL-7/CCL19 + anti-mouse PD1 antibody, a large number of T cell-positive cells were observed within the tumor and around the tumor.

The tumors of the mice administered with IL-7/CCL19, IL-7/CCL21, and IL-7/CCL19 + anti-mouse PD1 antibody were smaller; however, infiltration of CD8-positive T cells was observed even inside the tumors administered with BC-PIV alone (see FIG. 17). However, infiltration of lymphocytes in the periphery of the tumor was not observed in the administration of BC-PIV alone.

In the mice used in the current test, since BC-PIV is poorly permissive, the gene expression of the introduced IL-7, IL-12, CCL19, and CCL21 is assumed to be considerably low, but a high antitumor effect is still observed, while it is expected that expression would be higher in humans, which are permissive, and the antitumor effect would be even higher.

### [Experimental Example 10] Examination of antitumor effect of BC-PIV with cytokine (IL-7) and chemokine (CCL19) introduced therein on renal cancer RENCA cells and synergistic effect with anti-mouse PD1 antibody

Several kinds of systemic therapy using a tyrosine kinase inhibitor (TKI) and an immune checkpoint inhibitor (ICI), for example, an anti-PD1 antibody, have been carried out on patients with advanced renal cell carcinoma (aRCC), and the use of an ICI alone or in combination with another agent has been examined. This time, the inventors have examined the effects of an IL-7/CCL19-introduced BC-PIV and an anti-mouse PD1 antibody on RENCA cells, which are mouse renal cancer-derived cells.

Mouse RENCA renal cancer cells (8 × 10⁴ cells) other than CT26 cells were transplanted into the dorsal part of BALB/c mice, and when the tumor diameter reached 3 to 5 mm, the effects of a BC-PIV (Emp) without any exogenous gene introduced therein and an IL-7/CCL19-introduced BC-PIV within grown unilateral tumors were examined. This time, the synergistic effect of the immune checkpoint inhibitor with the anti-mouse PD1 antibody was also examined. It has been reported that in an in vitro test, the treatment with an anti-mouse PD1 antibody alone does not have an inhibitory effect on cell proliferation in RENCA cells (see Non Patent Document 2).

The group configuration of the mice included the following four groups.
(1): Untreated group (non-treat)
(2): BC-PIV group (Emp)
(3): BC-PIV/IL-7/CCL19 group (IL-7/CCL19)
(4): BC-PIV/IL-7/CCL19 + anti-mouse PD1 antibody group
(IL-7/CCL19 + αPD1)

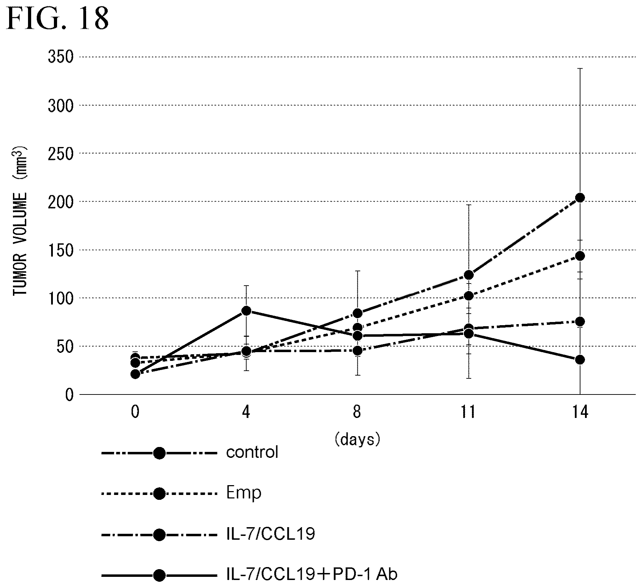

The results for the administered side are shown in FIG. 18. It is thought that the BC-PIV/IL-7/CCL19 and the BC-PIV/IL-7/CCL19 + anti-mouse PD1 antibody group are effective on the administered side. In the RENCA cells, the anti-mouse PD1 antibody alone does not exhibit an inhibitory effect on cell proliferation (see Non Patent Document 2), while an antitumor effect was observed when the anti-mouse PD1 antibody was used in combination with BC-PIV/IL-7/CCL19. Furthermore, an antitumor effect was also observed when BC-PIV/IL-7/CCL19 was administered alone. Although not shown in the drawing, on the virus-unadministered side, all tumors grew to approximately the same volume, and no antitumor effect was observed.

### [Experimental Example 11]

Antitumor effect of BC-PIV with cytokine (IL-12) introduced therein and synergistic effect with anti-mouse PD1 antibody

It has been found that IL-12 has extremely strong toxicity due to systemic immune activation, and the clinical effect is extremely limited even at the maximum tolerated dose (MTD). An antitumor effect was achieved by introducing IL-12 into BC-PIV and reducing the toxicity through local administration into the tumor. The IL-12 introduced this time is of human type, and the compatibility with mouse is hardly observed. However, in Non Patent Document 3, an antitumor effect of human IL-12 in mice has been reported. This time, the antitumor effect of human IL-12 introduced into BC-PIV was examined in mice.

The test was carried out by the same technique as the above-described method.

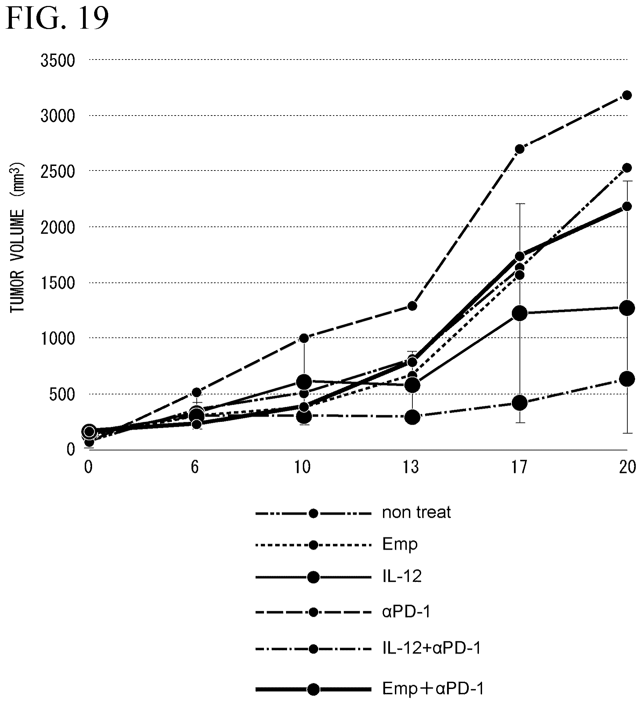

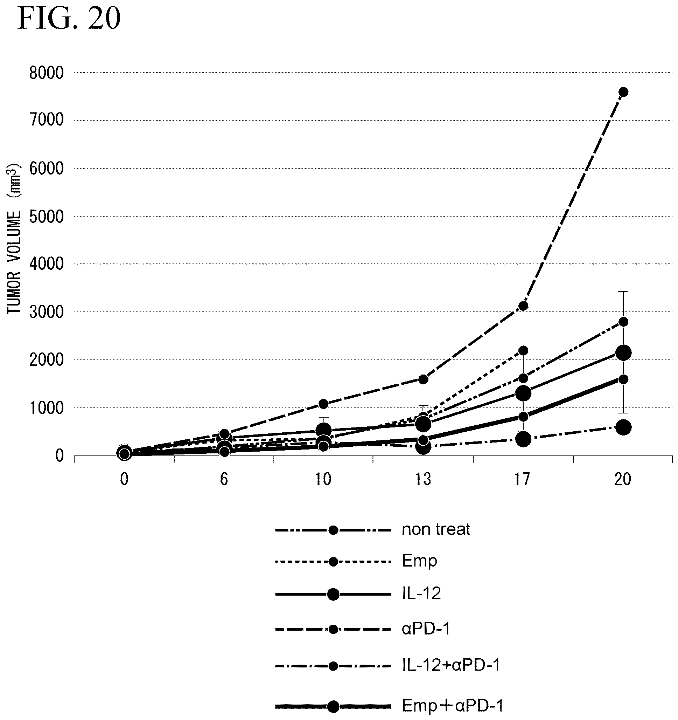

The antitumor effect of human IL-12 alone was observed on the administered side (see FIG. 19) and the non-administered side (see FIG. 20). When human IL-12 was administered together with an anti-mouse PD1 antibody, the antitumor effect was further enhanced (see FIG. 19 and FIG. 20).

### INDUSTRIAL APPLICABILITY

According to the present invention, an anticancer agent, a pharmaceutical composition for cancer treatment, a kit, and an activator, all of which have an excellent antitumor effect, can be provided.

## Claims

1. An anticancer agent for solid cancer, comprising, as an active ingredient, human parainfluenza virus type 2 expressing:
at least one kind of cytokine selected from the group consisting of IL-2, IL-7, IL-12, IL-15, and IL-18; or
a combination of the cytokine and CCL19 and/or CCL21; or
a mutant of either the cytokine or the combination, the mutant having the same function.

2. The anticancer agent for solid cancer according to Claim 1, wherein the at least one kind of cytokine is IL-12 constituting a heterodimer, or a mutant having the same function.

3. The anticancer agent for solid cancer according to Claim 1, wherein the human parainfluenza virus type 2 is of non-proliferative type in which an F gene is deleted from a genome.

4. A pharmaceutical composition for cancer treatment against solid cancer, the pharmaceutical composition comprising the anticancer agent for solid cancer according to any one of Claims 1 to 3.

5. The pharmaceutical composition for cancer treatment against solid cancer according to Claim 4, further comprising an immune activator and/or an immune checkpoint inhibitor.

6. The pharmaceutical composition for cancer treatment against solid cancer according to Claim 4, wherein the immune checkpoint inhibitor includes at least one kind selected from the group consisting of an anti-PD1 antibody, an anti-PD-L1 antibody, an anti-CTLA4 antibody, and an anti-TIM3 antibody.

7. The pharmaceutical composition for cancer treatment against solid cancer according to Claim 4, wherein the pharmaceutical composition for cancer treatment is used for intratumoral administration.

8. A kit for use in treatment of solid cancer, the kit comprising, for simultaneous use or sequential use in the treatment:
human parainfluenza virus type 2 expressing at least one kind of cytokine selected from the group consisting of IL-2, IL-7, IL-12, IL-15, and IL-18, or a combination of the cytokine and CCL19 and/or CCL21, or a mutant of either the cytokine or the combination, the mutant having the same function; and
an immune activator and/or an immune checkpoint inhibitor.

9. The kit according to Claim 8, wherein the at least one kind of cytokine is IL-12 constituting a heterodimer, or a mutant having the same function.

10. The kit according to Claim 8, wherein the human parainfluenza virus type 2 is of non-proliferative type in which an F gene is deleted from a genome.

11. The kit according to Claim 8, wherein the immune checkpoint inhibitor includes at least one kind selected from the group consisting of an anti-PD1 antibody, an anti-PD-L1 antibody, an anti-CTLA4 antibody, and an anti-TIM3 antibody.

12. The kit according to any one of Claims 8 to 11, wherein the kit is used for intratumoral administration.

13. A tumor-infiltrating T cell and dendritic cell activator, comprising, as an active ingredient:
human parainfluenza virus type 2 expressing at least one kind of cytokine selected from the group consisting of IL-2, IL-7, IL-12, IL-15, and IL-18; or a combination of the cytokine and CCL19 and/or CCL21; or a mutant of either the cytokine or the combination, the mutant having the same function.
